# EUROPÄISCHE PATENTSCHRIFT

(11) **EP 1 713 788 B1**
(45) Veröffentlichungstag und Bekanntmachung des Hinweises auf die Patenterteilung: **04.03.2009**
(21) Anmeldenummer: 05700995.3
(22) Anmeldetag: 18.01.2005
(51) Int. Cl.: C07D 333/14, C07D 333/20

(54) **VERFAHREN ZUR HERSTELLUNG VON ENANTIOMERENREINEN AMINOALKOHOLEN**
METHOD FOR PRODUCING ENANTIOMER-PURE AMINOALCOHOLS
PROCEDE POUR PREPARER DES ALCOOLS SOUS FORME D'ENANTIOMERES PURS

(30) Priorität: 29.01.2004 DE 102004004719
(43) Veröffentlichungstag der Anmeldung: 25.10.2006
(73) Patentinhaber: BASF SE, 67056 Ludwigshafen (DE)
(72) Erfinder: STÜRMER, Rainer, 67127 Rödersheim-Gronau (DE)
(86) Internationale Anmeldenummer: PCT/EP2005/000420
(87) Internationale Veröffentlichungsnummer: WO 2005/073215

(56) Entgegenhaltungen:
- EP-A- 1 300 405
- DE-A- 10 235 206
- DATABASE BEILSTEIN [Online] BEILSTEIN INSTITUT ZUR FÖRDERUNG DER WISSENSCHAFTEN; 19. Juli 2002 (2002-07-19), XP002333975 Database accession no. 9044800 & C. TANYELI ET AL.: ENANTIOMER, Bd. 6, Nr. 5, 2001, Seiten 259-266,
- A. KAMAL ET AL.: "Chemoenzymatic synthesis of duloxetin and its enantiomer: lipase-catalyzed resolution of 3-hydroxy-3-(2-thienyl)propannitril" TETRAHEDRON LETT., Bd. 44, 2003, Seiten 4783-4787, XP002333972
- S. SINGH ET AL.: "Chemoenzymatic synthesis of optically active heterocyclic homoallylic and homopropargylic alcohols" TETRAHEDRON: ASYMMETRY, Bd. 13, 2002, Seiten 2679-2687, XP002333973
- H. AKITA ET AL.: "Lipase catalyzed enantioselective hydrolysis of 2-methyl-3-acetoxy esters" TETRAHEDRON LETT., Bd. 27, Nr. 43, 1986, Seiten 5241-5244, XP002333974

## Beschreibung

Der Aminoalkohol **1** (Figur 1) [ (1S)-3-Methylamino-1-(2-thienyl)-propan-1-ol] ist ein gefragtes Zwischenprodukte bei der Herstellung des Pharmazeutikums Duloxetine^{®} ((+)-(S)-N-methyl-3-(1 naphthyloxy)-3-(2-thienyl)propylamin oxalat). Die bisherige Herstellmethode für dieses Intermediat ist aufwendig und benötigt teure und empfindliche Reagenzien. Ferner wird zur Herstellung einer reinen Verbindung eine technisch aufwendige Chromatographie benötigt, siehe beispielsweise EP 273658 A1; Liu et.al., Chirality 2000, 12(1), 26-29; Wheeler et.al, J. Labelled Comp. Radiopharm. 1995, 36(3), 213-23; US 5362886, EP 457559, Deeter et al, Tet. Lett. 1990, 31 (49), 7101-4

Es bestand daher die Aufgabe, einfachere und kostengünstigere Verfahren zur Herstellung von Duloxetine^{®} bzw. dessen Vorstufen bereitzustellen.

Die vorliegende Erfindung beschreibt einen neuen kostengünstigen Weg zu der isomerenreinen Verbindung. Ferner wird ein Weg beschrieben, um ein unerwünschtes Nebenprodukt ohne Chromatographie einfach abzutrennen.

Die erfindungsgemäße Synthese ausgehend vom Keton **3** [3-Chlor-1-(2-thienyl)-1-propanon] ist in Figur 1 gezeigt:
(i) Die Erfindung betrifft ein Verfahren zur Herstellung von enantiomerenreinem Alkohol der Formel **1,** wobei man
(ii) das Keton der Formel **3** zum racemischen Alkohol der Formel **4** reduziert,
(iii) den racemischen Alkohol der Formel **4** mit Bernsteinsäureanhydrid in Gegenwart einer Lipase enantioselektiv zum Bernsteinsäurehalbester der Formel **7** acyliert,
(iv) den Bernsteinsäurehalbester der Formel **7** vom nicht-umgesetzten Enantiomer der Formel **4** abtrennt,
(v) den enantiomerenreinen Alkohol der Formel **4** mit Methylamin zum enantiomerenreinen Alkohol der Formel **1** umsetzt.

Ausgehend vom Keton **3** [3-Chlor-1-(2-thienyl)-1-propanon] wird durch Reduktion, beispielsweise mit Borhydrid, bevorzugt NaBH₄, der entsprechende Alkohol **4** in racemischer Form gebildet (Stufe (i)). Bei dieser Reduktion tritt aber auch zu einem geringen Prozentsatz als Nebenprodukt der Alkohol 5 [1-(2-thienyl)-propan-1-ol] auf, der durch Hydrierung der Chlorfunktion entsteht. Das Nebenprodukt (Alkohol **5**) beträgt zwischen 1 und 15% des eingesetzten Ketons 3. Die Abtrennung dieses Alkohols **5** vom Produkt **4** auf dieser Stufe ist schwierig und erfordert eine aufwendige Chromatographie.

Vorteilhaft wird diese Verunreinigung in der letzten Stufe (Stufe (iv)) bei der Umsetzung mit Methylamin abgetrennt, da **5** nicht mit Methylamin reagiert und auch nicht kristallisiert.

In der nächsten Stufe (Stufe (ii)) wird der racemische Alkohol **4** mit Bernsteinsäureanhydrid in Gegenwart einer Lipase enantioselektiv zum Bernsteinsäurehalbester **7** acyliert, d.h. ein Enantiomer reagiert mit dem Bernsteinsäureanhydrid während das andere Enantiomer unverändert vorliegt. Die Selektivität der Acylierung kommt dabei durch die Stereospezifität der Lipase zustande.

In der Regel wird unter den beschriebenen Bedingungen das R-Enantiomer des Alkohols 4 zum Bernsteinsäurehalbester **7** acyliert, während das S-Enantiomer von **4** (= **6**) unverändert bleibt.

Für das erfindungsgemäße Verfahren geeignet sind eine Vielzahl von Lipasen, die in der organischen Synthese Verwendung finden (K. Faber, "Biotransformations in Organic Chemistry", Springer Verlag, Berlin, 2. Auflage 1995).

Besonders geeignete Lipasen sind solche der Homologiefamilie I.1 und I.2, wie sie gemäss Arpigny und Jäger (Biochem. J. 1999, 343, 177-183, Tabelle 1) klassifiziert werden. Hierunter sind besonders geeignet die Lipasen, die sich aus Pseudomonas-Stämmen, beispielsweise aus Pseudomonas aeruginosa, Pseudomonas fragi, Pseudomonas wisconsinensis, Pseudomonas fluorescens, Pseudomonas vulgaris, Pseudomonas luteola, Pseudomonas spec. DSM8246, aus Burkholderia-Stämmen, beispielsweise Burkholderia cepacia, Burkholderia glumae, Burkholderia plantarii, sowie aus Acinetobacter calcoaceticus, Chromobacterium viscosum, Vibrio cholerae isolieren lassen.

Ferner sind für das erfindungsgemäße Verfahren auch solche Lipasen geeignet, die ausgehend von den o.g. Lipasen durch genetic engineering verändert, adaptiert oder optimiert worden sind.

Die Lipasen können in Form ganzer Zellen, als zellfreier Extrakt oder in Form von aufgereinigten Proteinen eingesetzt werden. Besonders bevorzugt ist die Verwendung der Lipasen in Form von teilgereinigter oder hochgereinigter Proteinlösungen.

Die Lipasen können auch durch dem Fachmann bekannte Verfahren auf einem Träger immobilisiert und anschließend in dem erfindungsgemäßen Verfahren eingesetzt werden (z.B. Persson et al. Biotechnology Letters 2000, 22(19) 1571-1575). Die Verwendung von immobilisierten Lipasen ist insbesondere bei einer kontinuierlichen Verfahrensführung eine bevorzugte Ausführungsform. Hierzu können die Lipasen vorteilhaft immobilisiert in einer Säule oder einem Rohrreaktor verwendet werden.

Die Umsetzung (Stufe (ii)) geschieht bevorzugt in einem organischen Lösungsmittel, beispielsweise einem Kohlenwasserstoff oder einem Ester.

Besonders geeignete Lösungsmittel für die Umsetzung in Stufe (ii) sind aliphatische Kohlenwasserstoffe wie Hexan, Heptan, Octan oder Gemische davon. Weitere gut geeignete Lösungsmittel sind aromatische Kohlenwasserstoffe , insbesondere Benzol und Toluol. Gut geeignet sind ferner die niedrigen Alkylester der Kohlensäure wie Ethylencarbonat oder Propylencarbonat.

In einer weiteren Umsetzung (Stufe (iii)) wird nun der Bernsteinsäurehalbester (**7**) vom nicht umgesetzten Enantiomer (**6**) getrennt. Dies geschieht zweckmäßigerweise durch Extraktion des Bernsteinsäurehalbesters (**7**) bzw. dessen Salz, insbesondere dessen Alkalisalz, mittels einer wässrigen Phase. Eine bevorzugte Ausführungsform ist die in Beispiel 2 beschriebene wässrige Extraktion in Gegenwart von Natriumcarbonat.

Je nachdem welches Enantiomer des Alkohol **4** gewünscht wird, können nun entweder die organische Phase, die das Enantiomer **6** enthält oder die wässrige Phase, die das andere Enantiomer in Form des Bernsteinsäurehalbesters **7** enthält, aufgearbeitet werden. Der Bernsteinsäurehalbester **7** kann durch gängige Hydrolyseverfahren in Bernsteinsäure und das Enantiomer des Alkohols gespalten werden.

In einer weiteren Umsetzung (Stufe (iv)) wird der gewünschte enantiomerenreine Alkohol mit Methylamin zum enantiomerenreinen Aminoalkohol 1 umgesetzt.

Das geschieht üblicherweise mit Methylamin in Gegenwart von Wasser und Methanol, wie es in Beispiel 3 beschrieben wird. Auf dieser Stufe wird das bisher mitgeführte Nebenprodukt 5, das in Stufe (i) zu einem geringen Umfang entstanden ist, abgetrennt, da es nicht mit Methylamin reagiert und bei der anschließenden Reinigung von 1, beispielsweise durch Umkristallisieren, leicht entfernt werden kann.

Der durch das erfindungsgemäße Verfahren in hoher Enantiomerenreinheit darstellbare Aminoalkohol 1 ist ein gesuchtes Vorprodukt für die Herstellung des eingangs erwähnten Pharmazeutikums Duloxetine^{®}.

### Beispiel 1:

### Herstellung von 4 [3-Chlor-1-(2-thienyl)-propan-1-ol]:

In einem Gemisch aus 400 ml Toluol und 200 g Methanol werden 204 g (1.21 Mol) Keton **3** bei 0°C vorgelegt. Nach Zugabe von 2 g 30 % NaOH werden innerhalb von 2.5 h 21.4 g Natriumborhydrid portionsweise zugegeben. Nach 40 min Rühren bei 0°C wird das Reaktionsgemisch auf 500 ml eiskalte 10 % Essigsäure gegeben. Nach Abtrennen der wäßrigen Phase wird die organische Phase über Natriumsulfat trennen der wäßrigen Phase wird die organische Phase über Natriumsulfat getrocknet und vom Lösungsmittel befreit. Erhalten werden 198 g **4**, welches ca. 10 % **5** enthält.

### Beispiel 2:

### Herstellung von 6 [ (1S)-3-Chlor-1-(2-thienyl)-propan-1-ol]:

197 g (1.16 Mol) Alkohol **4**, enthaltend 10 % Alkohol **5** (d.i. das aus Beispiel 1 erhaltene Reaktionsprodukt) werden in 1 Liter MTBE vorgelegt. Dann werden 64.4 g (0.64 Mol) Bernsteinsäureanhydrid und 10 g Lipase aus Burkholderia plantarii zugegeben. Das Gemisch wird 20 h bei RT gerührt und durch Filtration vom Enzym befreit. Nach Zugabe von 1.4 l Wasser wird mit Natriumcarbonat auf pH = 9 gestellt und die Wässrige Phase abgetrennt. Die organische Phase wird über Natriumsulfat getrocknet und vom Lösungsmittel befreit. Es werden 96 g **6** erhalten, welches ca. 10 % des entsprechenden Antipoden von 5 enthält.

Beispiel 3:

### Herstellung von 1 [ (1S)-3-Methylamino-1-(2-thienyl)-propan-1-ol]

In einem Druckbehälter werden 20 g (0.093 Mol) Alkohol **6**, enthaltend ca. 10 % des entsprechenden Antipoden von **5** (d.i. das Reaktionsprodukt aus Beispiel 2) in 40 g Methanol vorgelegt und mit 74 g einer 40 % Methylamin Lösung in Wasser versetzt und 20 h bei 75°C unter Eigendruck gerührt. Nach beendigter Reaktion werden 5 g 30 % NaOH zugegeben und das Gemisch wird 10 min bei 80°C gerührt. Das Lösungsmittel wird entfernt und der Rückstand wird in 100 ml Toluol aufgenommen. Nach Abfiltrieren des unlöslichen Anteils wird der Rückstand aus Cyclohexan umkristallisiert.

Es wurden 13.17 g farblose Kristalle von **1** erhalten ( Drehwert : c= 1 in MeOH, alpha[D]25°C = -13.2° entsprechend einem optisch reinen Produkt.

Figur 1 beschreibt das Reaktionsschema für die Herstellung von (1 S)-3-Methylamino-1-(2-thienyl)-propan-1-ol ausgehend von 3-Chlor-1-(2-thienyl)-1-propanon

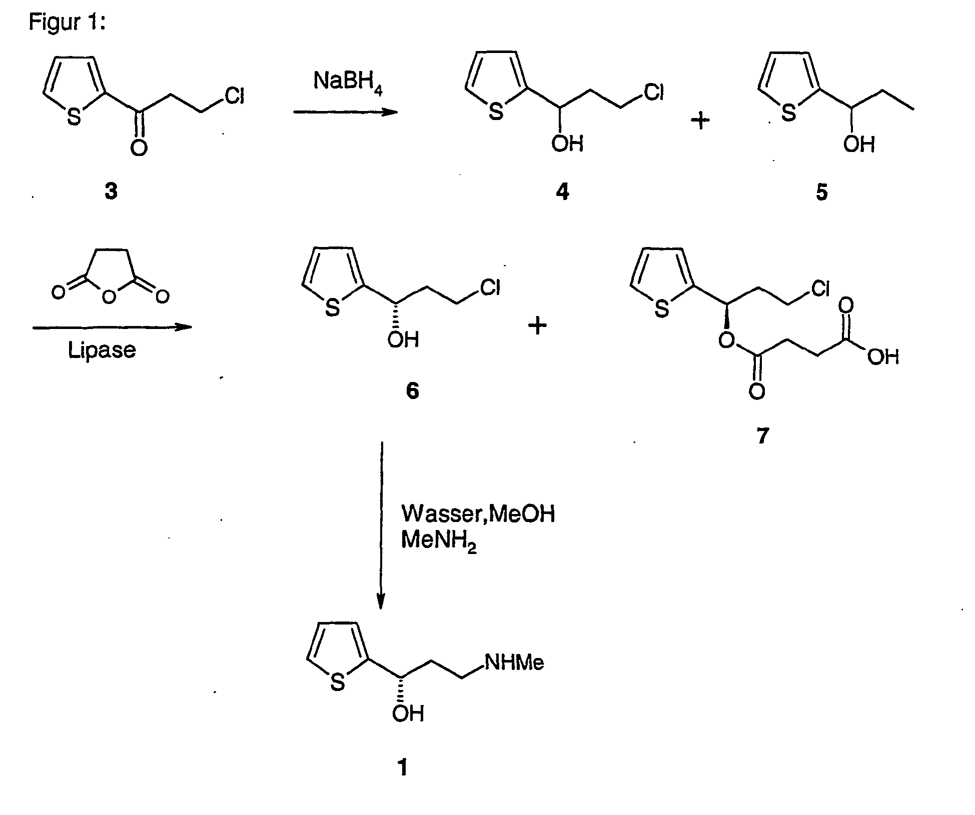

## Patentansprüche

1. Verfahren zur Herstellung von enantiomerenreinem Alkohol der Formel 1, wobei man 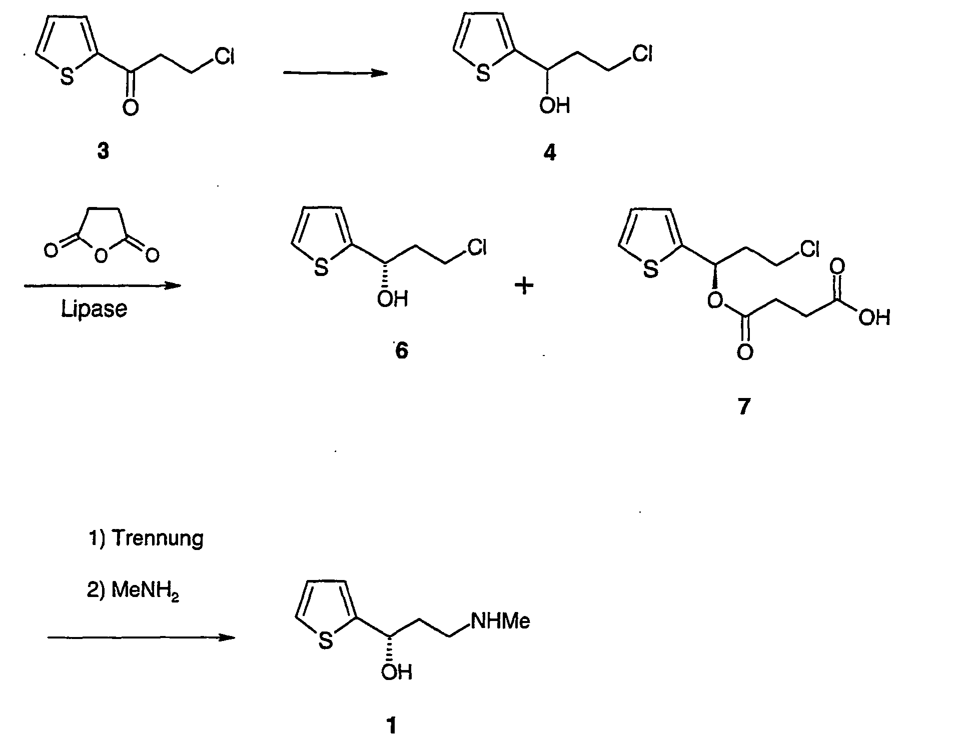
(i) das Keton der Formel **3** zum racemischen Alkohol der Formel **4** reduziert,
(ii) den racemischen Alkohol der Formel **4** mit Bernsteinsäureanhydrid in Gegenwart einer Lipase enantioselektiv zum Bernsteihsäurehalbester der Formel **7** acyliert,
(iii) den Bernsteinsäurehalbester der Formel **7** vom nicht-umgesetzten Enantiomer der Formel **4** abtrennt,
(iv) den enantiomerenreinen Alkohol der Formel **4** mit Methylamin zum enantiomerenreinen Alkohol der Formel **1** umsetzt.

2. Verfahren nach Anspruch 1, wobei die Reduktion in Stufe (i) mit NaBH₄ vorgenommen wird.

3. Verfahren nach Anspruch 1, wobei die Lipase in Stufe (ii) eine immobilisierte Lipase ist.

4. Verfahren nach Anspruch 1, wobei die Lipase in Stufe (ii) aus Burkholderia oder Pseudomonas ist.

5. Verfahren nach Anspruch 1, wobei die Abtrennung in Stufe (iii) in Form der konjugierten Base des Bernsteinsäurehalbesters der Formel **7** erfolgt.

6. Verfahren nach Anspruch 1, wobei die Umsetzung der Stufe (ii) in einem Kohlenwasserstoff als Lösungsmittel durchgeführt wird.

7. Verfahren nach Anspruch 6, wobei die als Lösungsmittel Heptan verwendet wird.

8. Verfahren nach Anspruch 1, wobei das Verfahren kontinuierlich betrieben wird.

9. Verfahren nach Anspruch 8, wobei eine immobilisierte Lipase in einem Säulenreaktor verwendet wird.

10. Verfahren nach Anspruch 9, wobei als Lösungsmittel in Stufe (ii) Ethylencarbonat oder Propylencarbonat verwendet wird.

## Claims

1. A process for preparing enantiomerically pure alcohol of the formula 1, which comprises 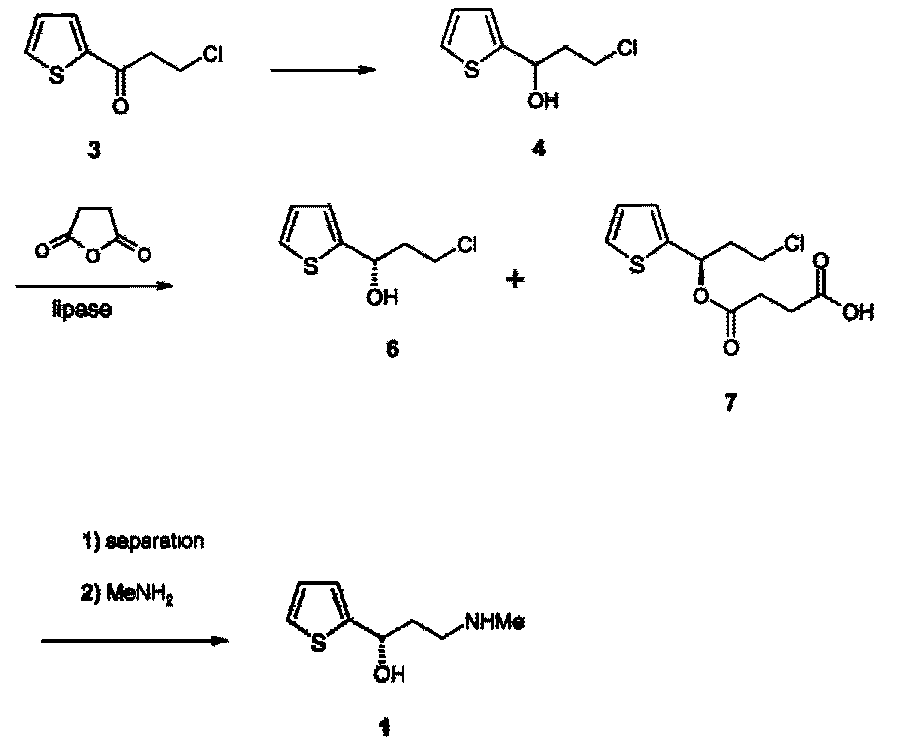
(i) reducing the ketone of the formula **3** to the racemic alcohol of the formula 4,
(ii) enantioselectively acylating the racemic alcohol of the formula **4** with succinic anhydride in the presence or a lipase to give the succinic semiester of the formula **7**,
(iii) separating off the succinic semiester of the formula **7** from the unreacted enantiomer of the formula 4,
(iv) reacting the enantiomerically pure alcohol of the formula **4** with methylamine to give the enantiomencally pure alcohol of the formula **1**

2. The process according to claim 1, wherein the reduction in step (i) is performed using NaBH₄.

3. The process according to claim 1, wherein the lipase in step (ii) is an immobilized lipase.

4. The process according to claim 1, wherein the lipase in step (ii) is derived from Burkholderia or Pseudomonas.

5. The process according to claim 1, wherein the separation in step (iii) takes place in the form of the conjugated base of the succinic semiester of the formula **7**.

6. The process according to claim 1, wherein the reaction of step (ii) is carried out in a hydrocarbon as solvent

7. The process according to claim 6, wherein heptane is used was the solvent.

8. The process according to claim 1, wherein the process is operated continuously.

9. The process according to claim 8, wherein an immobilized lipase is used In a column reactor.

10. The process according to claim 9, wherein ethylene carbonate or propylene carbonate is used as the solvent in step (ii).

## Revendications

1. Procédé de fabrication d'un alcool énantiomériquement pur de formule 1, selon lequel 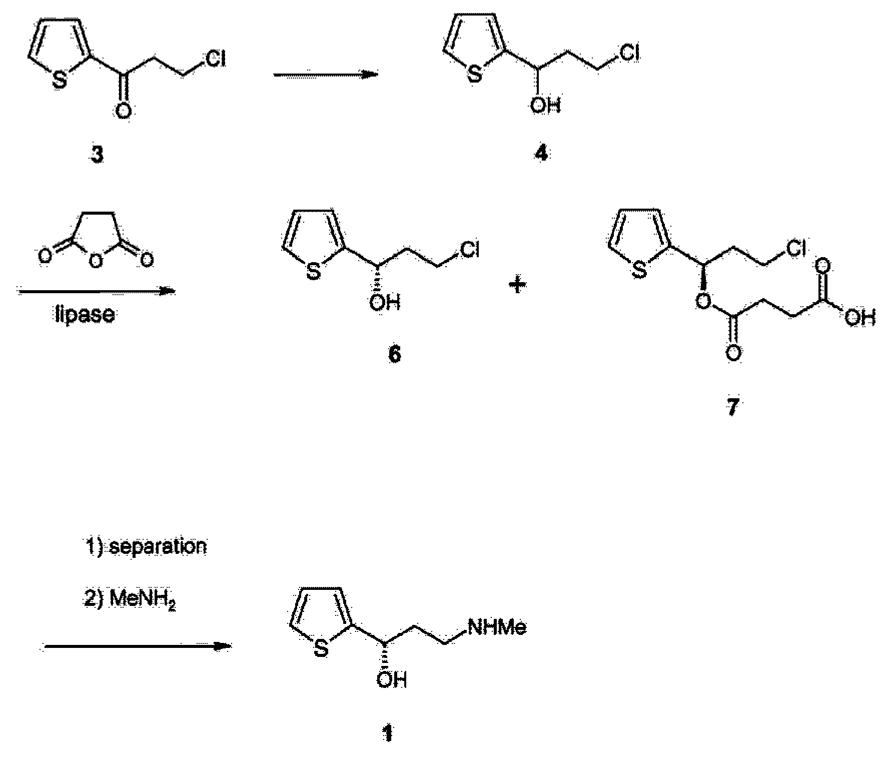
(i) la cétone de formule 3 est réduite en alcool racémique de formule 4,
(ii) l'alcool racémique de formule 4 est acylé de manière énantiosélective avec l'anhydride de l'acide succinique en présence d'une lipase pour former le semi-ester de l'acide succinique de formule 7,
(iii) la semi-ester de l'acide succinique de formule 7, est séparé des énantiomères non réagis de formule 4,
(iv) l'alcool énantiomériquement pur de formule 4 est mis en réaction avec la méthylamine pour former l'alcool énantiomériquement pur de formule 1.

2. Procédé selon la revendication 1, dans lequel la réduction a l'étape (i) est réalisée avec du NaBH₄.

3. Procédé selon la revendication 1, dans lequel la lipase de l'étape (ii) est une lipase immobilisée.

4. Procédé selon la revendication 1, dans lequel la lipase de l'étape (ii) est issue de Burkholderia ou de Pseudomonas.

5. Procédé selon la revendication 1, dans lequel la séparation à l'étape (iii) a lieu sous forme de la base conjuguée du semi-ester de l'acide succinique de formule 7.

6. Procédé selon la revendication 1, dans lequel la réaction à l'étape (ii) est réalisée dans un hydrocarbure en tant que solvant.

7. Procédé selon la revendication 6, dans lequel l'heptane est utilisé en tant que solvant.

8. Procédé selon la revendication 1, dans lequel le procédé fonctionne de manière continue.

9. Procédé selon la revendication 8, dans lequel une lipase immobilisée dans une colonne de réaction est utilisée.

10. Procédé selon la revendication 9, dans lequel du carbonate d'éthylène ou du carbonate de propylène est est utilisé en tant que solvant à l'étape (ii).
